(19) Europäisches Patentamt
European Patent Office
Office européen des brevets

(11) **EP 1 138 321 B1**

(12) **EUROPÄISCHE PATENTSCHRIFT**

(45) Veröffentlichungstag und Bekanntmachung des Hinweises auf die Patenterteilung:
**03.01.2007 Patentblatt 2007/01**

(51) Int Cl.:
***A61K 9/22*** *(2006.01)*

(21) Anmeldenummer: **01105547.2**

(22) Anmeldetag: **06.03.2001**

(54) **Feste orale Darreichungsformen mit retardierter Wirkstofffreisetzung und hoher mechanischer Stabilität**

Solid oral dosage forms with sustained drug release and high mechanical stability

Formes d'administration orale solides à libération retardée du principe actif et dotées d'une stabilité mécanique élevée

(84) Benannte Vertragsstaaten:
**AT BE CH CY DE DK ES FI FR GB GR IE IT LI LU MC NL PT SE TR**
Benannte Erstreckungsstaaten:
**RO SI**

(30) Priorität: **29.03.2000 DE 10015479**

(43) Veröffentlichungstag der Anmeldung:
**04.10.2001 Patentblatt 2001/40**

(73) Patentinhaber: **BASF Aktiengesellschaft**
**67056 Ludwigshafen (DE)**

(72) Erfinder:
• **Kolter, Karl, Dr.**
  **67117 Limburgerhof (DE)**
• **Schönherr, Michael, Dr.**
  **67227 Frankenthal (DE)**
• **Ascherl, Hermann**
  **67246 Dirmstein (DE)**

(56) Entgegenhaltungen:
EP-A- 0 231 826        EP-A- 0 575 930
EP-A- 1 138 320        DE-A- 19 709 663
US-A- 3 458 622

**Beschreibung**

Die vorliegende Erfindung betrifft nicht schwimmfähige feste orale Darreichungsformen mit retardierter Wirkstofffreisetzung bei gleichzeitig hoher mechanischer Stabilität, enthaltend neben einer vorformulierten Mischung aus Polyvinylacetat und Polyvinylpyrrolidon noch weitere wasserlösliche Polymere oder lipophile Zusatzstoffe.

Retardierte Darreichungsformen gewinnen eine immer größere Bedeutung, zum einen, weil die Verabreichungshäufigkeit reduziert werden kann und zum anderen, weil sie zu einer Reduzierung der Blutspiegelschwankungen führen. Der kleinere maximale Blutspiegel kann die Schwere von dosisabhängigen Nebenwirkungen verringern und so beispielsweise bei Arzneimitteln die Verträglichkeit verbessern. Die größere minimale Plasmakonzentration erhöht die Wirksamkeit besonders von Wirkstoffen, bei denen eine bestimmte Konzentrationsschwelle nicht unterschritten werden sollte.

Die gewünschte protahiert-kontrollierte Wirkstofffreigabe kann durch die Einbettung des Wirkstoffes in eine inerte Matrix erfolgen. Solche (Retard-)Matrixtabletten werden in der Regel durch Direktverpressung der entsprechenden Pulvermischung oder durch vorgeschaltete Granulation mit anschließender Komprimierung hergestellt. Eine weitere Möglichkeit zur Herstellung einzeldosierter fester Matrix-Formlinge bietet das Extrusionsverfahren. Nach dem Verpressen oder Extrudieren bildet die Plastmasse ("Matrixretardierungsmittel") den porösen zusammenhängenden Gerüstkörper in dem der (die) Wirkstoff(e) homogen verteilt vorliegen. An die dafür geeigneten Gerüstbildner ("Matrixretardierungsmittel") stellt man die für die entsprechende Verarbeitungstechnologie (v.a. Direkttablettierung) notwendigen physikalisch-chemischen Anforderungen, u.a. gute Fließfähigkeit und gute Verpreßbarkeit.

Das Verfahren der Direkttablettierung stellt einen vergleichsweise einfachen, preiswerten und zeitsparenden Prozeß insbesondere bei der Arzneiformherstellung dar und bietet dadurch der pharmazeutischen Industrie viele Vorteile. Zudem lassen sich durch die Direkttablettierung auch hitze- und/oder feuchtigkeitsempfindliche Wirkstoffe verarbeiten.

Generelle Anforderungen an einen Hilfsstoff für die Herstellung von direkttablettierbaren Retardmatrixtabletten sind demnach:

- gute Fließfähigkeit

- hohe plastische Verformbarkeit

- geringe Entmischungstendenz in der Tablettiermischung

- Ausbildung einer ausreichend mechanisch stabilen Matrix für Lagerung, Transport und Anwendung

- gutes Retardierungspotential

- Retardierung unabhängig von pH-Wert, Ionenstärke, mechanischer Beanspruchung

- inertes Verhalten gegenüber sämtlichen Wirkstoffen

Hydroxypropylmethylcellulose (Methocel®), der bisher am häufigsten für Matrixtabletten eingesetzte Hilfsstoff, zeigt den deutlichen Nachteil einer schlechten Fließfähigkeit, einer niedrigen Plastizität und einer schlechten Verpreßbarkeit.

Andere zur Matrixretardierung übliche Hilfsstoffe sind z.B. Hydroxypropylcellulose, Xanthan und Alginsäure. Insgesamt sind bei Einsatz der bisher zur Matrixretardierung üblichen Hilfsstoffe folgende Probleme offensichtlich:

- schlechte Fließfähigkeit

- schlechte Verpreßbarkeit

- Klebeneigung

- Ungünstige Beeinflussung des Wirkstofffreisetzungsprofils durch Einflüsse wie pH-Wert, Ionenstärke und mechanische Beanspruchung usw.

- Chargenvariabilität mit einhergehender Änderung der Produkteigenschaften insbesondere bei Produkten natürlichen Ursprungs.

Tabletten sollten mechanisch sehr stabil sein, weil sonst Abrieb und Bruch während der weiteren Verarbeitung, z.B. bei Coating und Verpackung, entstehen.

Die Beurteilung der Erosionsstabilität für Matrixretardformulierungen ist insofern von Bedeutung, da z.B. die Peristaltik des Magen- und Darmtraktes die Freigabecharakteristik maßgeblich beeinflussen kann. Gerade bei Methocel® mit quellungskontrollierter Retardierungsmatrix könnten beispielsweise durch Reibung mit Nahrungsbestandteilen die gequollenen Polymerschichten unkontrollierbar abgeschliffen werden, was einer kontrollierten Matrixretardierung widerspricht. Eine in-vitro - in-vivo Korrelation ist so äußerst fraglich.

Inerte Matrixbildner wie z.B. Ethylcellulose, Ammoniummethacrylat-Copolymer (Eudragit® RS oder RL), Stearylalkohol und Stearinsäure zeigen ebenfalls zahlreiche Nachteile wie schlechte Fließfähigkeit, schlechte Verpreßbarkeit, Klebeneigung, Beeinflussung der Wirkstofffreisetzung durch pH-Wertänderungen und Chargenvariabilität. Hinzu kommt, daß durch die hohe Lipophilie einiger dieser Stoffe Wirkstoffe in der Matrix zum Teil vollständig eingeschlossen werden, wodurch keine vollständige Freisetzung der gesamten Dosis erfolgt. Dies ist insbesondere bei der Verwendung für Arzneimittel nicht akzeptabel.

Die formulierte Mischung aus Polyvinylacetat und Polyvinylpyrrolidon ist, da es sich um eine innige Verbindung eines lipophilen mit einem hydrophilen Polymer handelt, für die Retardierung besser als die oben genannten Stoffe geeignet. Solche Kombinationen sind beschrieben in US-Patent 5,490,990.

Aus der EP-A 1 138 320 sind wirkstoffhaltige Schwimmformen bekannt, die eine formulierte Mischung aus Polyvinylacetat und Polyvinylpyrolidon enthalten.

Aus der EP-A 0 231 826 sind theophyllinhaltig Retardtabletten bekannt, die neben Celluloseacetatphthalat als Hilfsstoffe Polyvinylacetat und Polyvinylpyrrolidon enthalten. Dabei wird der Wirkstoff mit Celluloseacetatphthalat und Polyvinylpyrrolidon granuliert und dieses Granulat mit Polyvinylacetat, Polyvinylpyrrolidon und einem Schmiermittel gemischt und zu Tabletten verarbeitet.

Arzneistoffmatrices auf Basis einer formulierten Mischung aus Polyvinylacetat und Polyvinylpyrrolidon bilden während der Magen-Darm-Passage nach und nach feine Poren, durch die der Arzneistoff langsam hinausdiffundiert. Die inerte, wirkstofffreie Hilfsstoffmatrix wird dann unverändert mit den Faeces ausgeschieden. Damit geschieht die Freigabe des Wirkstoffes weitgehend unabhängig von äußeren Faktoren wie Füllungsgrad des Magens, Darmmotilität usw.

Die diffusionskontrollierte Freigabe aus solchen Matrices kann mathematisch mit folgender Gleichung beschrieben werden:

$$Q = \sqrt{\frac{D \cdot \varepsilon}{\tau} - (2 \cdot A - \varepsilon \cdot C_S) \cdot C_S} \cdot \sqrt{t}$$

Die formulierte Mischung aus Polyvinylacetat und Polyvinylpyrrolidon vereint eine hohe mechanische Stabilität bei gleichzeitiger guter Retardierung. Die exzellenten Fließeigenschaften und die hohe Plastizität ermöglichen eine Verarbeitung von ansonsten kritischen Tablettiermischungen. Als synthetisches Produkt entfallen selbstverständlich die Nachteile eines Naturproduktes, wie schwankende Produktqualitäten durch Chargeninhomogenität.

Die Einstellung der Wirkstofffreisetzung muß prinzipiell bei jedem Wirkstoff individuell erfolgen, da sie sich an den pharmakologischen, biochemischen und physiko-chemischen Eigenschaften des Wirkstoffes und der gewünschten Wirkdauer orientieren muß. Es ist bekannt, daß die Wirkstofffreisetzung durch eine Erhöhung oder Erniedrigung des Anteils an der formulierten Mischung aus Polyvinylacetat und Polyvinylpyrrolidon verändert werden kann. Bei verschiedenen Wirkstoffen führt diese Variationsmöglichkeit nicht zu einem befriedigenden Ergebnis. Außerdem verändert sich dabei immer die Größe einer Tablette.

Bei schwerlöslichen Wirkstoffen würde eine recht geringe Menge an der formulierten Mischung aus Polyvinylacetat und Polyvinylpyrrolidon zur Retardierung ausreichen, jedoch sind die Schwankungen in der Freisetzung von Tablette zu Tablette recht groß, da der Gerüstaufbau Zufälligkeiten unterworfen ist und die mechanische Stabilität der Tablette schlecht ist. Die Einarbeitung einer größeren Menge an der formulierten Mischung aus Polyvinylacetat und Polyvinylpyrrolidon wäre wünschenswert.

Bei anderen Wirkstoffen stört die etwas zu schnelle anfängliche Freisetzung, da bei nicht quellenden Gerüstmatrices die Freisetzung nach dem "Wurzel-t-Gesetz" erfolgt. Es wäre daher wünschenswert, diese schnelle anfängliche Freisetzung durch Zumischungen zur Tablettenrezeptur zu verringern, ohne daß die Vorteile der Polyvinylacetat - Polyvinylpyrrolidon - Matrix verlorengehen.

Bei sehr leicht wasserlöslichen Wirkstoffen ist die Freisetzung aus einer Polyvinylacetat - Polyvinylpyrrolidon - Matrix oft recht schnell, bzw. es werden große Mengen an Polyvinylacetat - Polyvinylpyrrolidon benötigt, die die Form stark vergrößern und schwer schluckbar machen. Solche Stoffe können bisher mit diesem Retardierungsmittel nur schlecht retardiert werden.

Möglichkeiten zur Einstellung dieser Freisetzungsprofile unter Erhalt des Gerüstes und der mechanischen Stabilität sind bisher nicht bekannt.

Aufgabe der vorliegenden Erfindung war es daher, eine feste orale Darreichungsform mit retardierter Wirkstofffreisetzung bei gleichzeitig hoher mechanischer Stabilität zu entwickeln.

Die Aufgabe wurde erfindungsgemäß gelöst durch nicht schwimmfähige orale Darreichungsformen mit retardierter wirkstofffreisetzung und hoher mechanischer Stabilität enthaltend

(a) einen oder mehrere Wirkstoffe

(b) eine formulierte Mischung aus Polyvinylacetat und Polyvinylpyrrolidon

(c) wasserlösliche Polymere oder nieder- oder hochmolekulare lipophile Zusatzstoffe

(d) sowie weitere, übliche Hilfsstoffenwobei das Verhältnis Polyvinylacetat und Polyvinylpyrrolidon 6:4 bis 9:1 beträgt.

Bevorzugt werden die Darreichungsformen für pharmazeutische Wirkstoffe eingesetzt. Sie können aber auch für jeden anderen Wirkstoff, bei dem eine verzögerte Freisetzung erwünscht ist eingesetzt werden.

Durch den Zusatz an wasserlöslichen Polymeren oder lipophilen Zusatzstoffen kann die Freisetzung in fast beliebigen Grenzen variiert werden, bei gleichzeitig guter Fließfähigkeit der Tablettiermischung, hoher Bruchfestigkeit und niedrigem Abrieb der Tabletten. Durch den Zusatz von niedrigviskosen, nichtquellenden wasserlöslichen Polymeren, wie Polyvinylalkohole, Polyethylenglykole, Polyoxyethylen-Polyoxypropylen-Blockpolymerisate, Polyvinylpyrrolidone sowie Derivate, Vinylacetat-Vinylpyrrolidon-Copolymere, vorzugsweise Polyethylenglykolen, Polyvinylpyrrolidonen, Vinylacetat-Vinylpyrrolidon-Copolymeren oder Maltodextrinen, kann die Wirkstofffreisetzung beschleunigt werden.

Diese Zusatzstoffe werden in Konzentrationen von 1 bis 40 %, bevorzugt von 2 bis 30 % bezogen auf das Tablettengesamtgewicht eingesetzt. Dies ist erforderlich bei sehr niedrig dosierten Wirkstoffen, wo die zum Gerüstaufbau erforderliche Menge an formulierter Mischung aus Polyvinylacetat und Polyvinylpyrrolidon eine zu starke Retardierung mit sich bringt. Ferner auch bei schwerlöslichen Wirkstoffen, bei denen niedrige Mengen an Retardierungsmittel zwar zu einer verzögerten Freisetzung führen, aber der Gerüstaufbau unvollständig ist, starken Schwankungen unterliegt und die mechanische Stabilität der Tabletten unzureichend ist. Dies ist insbesondere der Fall wenn der Wirkstoff schlecht preßbar ist.

Auch die schlechte Fließfähigkeit des Wirkstoffes kann dann durch die geringe Menge an formulierter Mischung aus Polyvinylacetat und Polyvinylpyrrolidon nicht entscheidend verbessert werden. Die Erhöhung des Anteils an Retardierungsmittel verbessert diese Eigenschaften, führt dann aber zu einer zu stark retardierten Freisetzung. Das wasserlösliche nicht quellende Polymer beschleunigt die Freisetzung und stabilisiert diese gegen äußere Einflüsse. Auch die Reproduzierbarkeit ist sehr viel besser. Die üblichen Tablettierhilfsstoffe wie Lactose, Calciumphosphate, Sorbit, Mannit, mikrokristalline Cellulose oder Stärke sind hierzu nicht oder nicht ausreichend in der Lage. Wahrscheinlich führt eine Wechselwirkung des wasserlöslichen Polymers mit einer formulierten Mischung aus den Polymeren Polyvinylacetat und Polyvinylpyrrolidon zu der sehr stabilen, preßdruckunabhängigen und reproduzierbaren Freisetzung. Auch die Bruchfestigkeit der Tabletten und der Abrieb zeigen ausgezeichnete Werte, oft sogar höher als ohne Zumischung wasserlöslicher Polymere.

Der Abrieb sollte kleiner 3 %, bevorzugt kleiner 1,5 %, besonders bevorzugt kleiner 1 % liegen.

Wasserlösliche aber quellende, hochviskose Polymere führen überraschenderweise zu einer langsameren Freisetzung. Es wäre zu erwarten gewesen, daß das inerte Gerüst durch das quellende Polymer zerstört wird und der Wirkstoff schneller freigesetzt wird. Daß dies nicht eintritt liegt wahrscheinlich an der großen Elastizität der formulierten Mischung aus Polyvinylacetat und Polyvinylpyrrolidon. Die sich in den Poren des Gerüstes bildende hochviskose Lösung aus dem wasserlöslichen, quellenden Polymer blockiert diese und verlangsamt so die Diffusion des Wirkstoffes nach außen. Die Freisetzung ist häufig stärker verlangsamt als durch die beiden Komponenten alleine. Es ist eine synergistische Wirkung vorhanden. Hinzu kommt, daß auch die initiale Freisetzung durch eine Gelbildung an der Oberfläche reduziert wird und das Freisetzungsprofil dadurch "linearisiert" wird. Die mechanischen Eigenschaften der Tabletten bleiben auf einem sehr hohen Niveau.

Als wasserlösliche quellende Polymere können eingesetzt werden: Alginate, Pektine, Galactomannane, Carrageenane, Dextran, Curdlan, Pullulan, Gellan, Chitin, Gelatine, Xanthane, Hemicellulosen, Cellulosederivate wie Methylcellulose, Hydroxypropylmethylcellulose, Hydroxypropylcellulose, Hydroxyethylcellulose, Methylhydroxyethylcellulose, Carboxymethylcellulose, Stärkederivate wie Carboxymethylstärke, abgebaute Stärke, Polyacrylsäure, Polymethacrylsäure, Acrylsäure-Methacrylsäure-Copolymere. Mögliche Salze dieser Stoffe sind ebenfalls eingeschlossen.

Diese Zusatzstoffe werden in Konzentrationen von 1 bis 40 %, bevorzugt von 2 bis 30 % bezogen auf das Tablettengewicht eingesetzt.

Eine Verstärkung der Retardwirkung kann auch durch feinteilige lipophile Zusatzstoffe erfolgen. Hierbei lagern sich diese Zusatzstoffe in die Poren und Kanäle des Gerüstes aus Polyvinylacetat und Polyvinylpyrrolidon und blockieren diese. Es ist wichtig, daß diese Stoffe in kleiner Korngröße eingesetzt werden, da sie in grober Form keine bzw. nur eine geringe Wirkung entfalten. Als lipophile Zusatzstoffe können sowohl Polymere als auch niedermolekulare Verbindungen verwendet werden. Bevorzugt sind allerdings die Polymere.

Zu diesen Zusatzstoffen zählen: Cellulosederivate wie Ethylcellulose, Celluloseacetat, Celluloseacetatphthalat, Celluloseacetatsuccinat, Hydroxypropylmethylcelluloseacetatphthalat, Hydroxypropylmethylcelluloseacetatsuccinat, Acrylatester-Methacrylatester-Copolymerisate insbesondere Methylmethacrylat-Ethylacrylat-Copolymere, Ammonio-Methacrylate-Copolymer Typ A und Typ B, Methacrylsäure-Acrylsäureester-Copolymere insbesondere Methacrylsäure-Ethylacrylat-Copolymere, Fettalkohole wie Stearylalkohol, Fettsäuren wie Stearinsäure, Fettsäureester und Fettalkoholester, Glyceride, Wachse, Lecithin.

Diese Zusatzstoffe werden in Konzentrationen von 1 bis 40 %, bevorzugt von 2 bis 30 % bezogen auf das Tablettengesamtgewicht eingesetzt.

In den erfindungsgemäßen Zubereitungen liegt die formulierte Mischung aus Polyvinylacetat und Polyvinylpyrrolidon in Konzentrationen von 10 bis 80 %, vorzugsweise von 20 bis 60 % vor.

Das Verhältnis von Polyvinylacetat und Polyvinylpyrrolidon in der formulierten Mischung liegt zwischen 6:4 bis 9:1. Nicht in diesem Bereich liegende Verhältnisse zeigen nicht den gewünschten Effekt in Bezug auf Retardierung und mechanische Eigenschaften.

Die erfindungsgemäßen Darreichungsformen umfassen orale Darreichungsformen wie Tabletten, Extrudate, Pellets oder Granulate.

Sie können mittels Direktverpressung, Extrusion, Schmelzextrusion, Pelletierung oder Kompaktierung hergestellt werden.

Auch Trockengranulationsprozesse und Feuchtgranulationsprozesse können verwendet werden.

Kleinere Formlinge wie beispielsweise Pellets oder Mikrotabletten können auch in Kapseln eingebracht werden.

Natürlich können auch weitere übliche Tablettierhilfsstoffe beispielsweise Bindemittel, Streckmittel/Füllstoffe, Sprengmittel, Schmiermittel, Fließmittel, Farbstoffe, Stabilisatoren wie Antioxidantien, Netzmittel, Konservierungsmittel, Formentrennmittel, Aromen und Süßstoffe eingesetzt werden.

Als Schmiermittel können Stearate von Aluminium, Calcium, Magnesium und Zinn, sowie Magnesiumsilikat, Silikone und ähnliche verwendet werden.

Fließmittel können beispielsweise sein, Talk oder kolloidales Siliciumdioxid.

Bindemittel sind z.B. mikrokristalline Cellulose.

Sprengmittel können sein quervernetztes Polyvinylpyrrolidon oder quervernetzte Natriumcarboxymethylstärke. Stabilisatoren können sein Ascorbinsäure oder Tocopherol.

Als Füllstoffe können z.B. anorganische Füllstoffe wie Oxide von Magnesium, Aluminium, Silicium, Titan- oder Calciumcarbonat, Calcium- oder Magnesiumphosphate oder organische Füllstoffe wie Lactose, Saccharose, Sorbit, Mannit zugesetzt werden.

Farbstoffe sind z.B. Eisenoxide, Titandioxid, Triphenylmethanfarbstoffe, Azofarbstoffe, Chinolinfarbstoffe, Indigotinfarbstoffe, Carotinoide, um die Darreichungsformen einzufärben, Opakisierungsmittel wie Titandiodid oder Talkum, um die Lichtdurchlässigkeit zu erhöhen und um Farbstoffe einzusparen.

Die erfindungsgemäßen Darreichungsformen können jeden Wirkstoff für den eine verzögerte Freisetzung erwünscht ist enthalten.

Bevorzugt werden als Wirkstoffe Nahrungsergänzungs- oder Zusatzstoffe, Vitamine, Mineralstoffe oder Spurenelemente, insbesondere bevorzugt aber pharmazeutische Wirkstoffe eingesetzt.

Pharmazeutische Formulierungen der oben genannten Art können durch Verarbeiten der beanspruchten Verbindungen mit pharmazeutischen Wirkstoffen nach herkömmlichen Methoden und unter Einsatz bekannter und neuer Wirkstoffe erhalten werden. Die Wirkstoffe können dabei aus jedem Indikationsgebiet kommen.

Als Beispiele seien hier die folgenden genannt:

Benzodiazepine, Antihypertensiva, Vitamine, Cytostatika, Anästhetika, Neuroleptika, Antidepressiva, Antibiotika, Antimykotika, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Durchblutungsfördernde Mittel, Diuretika, Diagnostika, Corticoide, Cholinergika, Gallenwegstherapeutika, Antiasthmatika, Broncholytica, Betarezeptorenblocker, Calciumantagonisten, ACE-Hemmer, Arteriosklerosemittel, Antiphlogistika, Antikoagulatia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel.

Die Tablettenform kann in weiten Grenzen variiert werden. So sind gewölbte, biplane, runde, kantige Tabletten herstellbar wie auch oblong- oder football-shape-Formen. Die Größe wird nach oben limitiert durch die Schluckbarkeit nach unten durch maschinenbauliche Grenzen. Übliche Tablettengrößen liegen zwischen 1 und 16 mm, vorzugsweise zwischen 2 und 13 mm Durchmesser.

Daneben lassen sich auch Zwei- oder Mehrschichttabletten herstellen, bei denen eine Schicht die gesamte Dosis an Wirkstoff enthält oder zumindest sehr wirkstoffreich ist, während die andere Schicht sehr reich ist an der Kombination Polyvinylacetat-Polyvinylpyrrolidon. Dadurch kann zusätzlich die Wirkstofffreisetzung gezielt beeinflußt werden. Es ist sogar möglich, unter Verwendung von zwei oder mehreren Wirkstoffen diese mit unterschiedlichen Geschwindigkeiten freizusetzen, indem sie völlig oder zum größten Teil getrennt in einzelne Schichten eingearbeitet werden.

Eine besondere Ausprägung ist die Herstellung von Manteltabletten, bei denen der Kern sehr wirkstoffreich ist bzw. sogar die Gesamtmenge an Wirkstoff enthalten kann, während die Hülle zu einem großen Teil aus der Kombination Polyvinylacetat-Polyvinylpyrrolidon besteht. Dadurch wird eine starke Retardierung erzeugt. Diese Form ist besonders für sehr leicht wasserlösliche Wirkstoffe, die stark retardiert werden sollen, geeignet.

Die erfindungsgemäßen Tabletten können auch durch Schmelzextrusion und anschließende Kalandrierung hergestellt werden.

Die Tabletten können in üblicher Weise mit einem Filmüberzug versehen werden. Dieses Coating kann wasserlöslich sein, dann dient es lediglich der Verbesserung des optischen Erscheinungsbildes bzw. der Überdeckung eines schlechten Geruches oder Geschmacks, es kann aber auch wasserunlöslich sein und dann zur weiteren Verringerung der Wirkstofffreisetzung verwendet werden. Dies ist notwendig, wenn eine sehr lange Wirkdauer angestrebt wird. Prinzipiell sind alle pharmazeutisch zugelassenen Coatingmaterialien einsetzbar, beispielsweise Hydroxypropylmethylcellulose (Pharmacoat 603 oder 606, Fa. Shin-Etsu), Hydroxypropylcellulose, Ethylcellulose, Celluloseacetatphthalat, Ammoniomethacrylat Copolymer (USP), Methacrylsäure Copolymer Typ C (USP), Butylmethacrylat-2-dimethylaminoethylmethacrylatmethylmethacrylat-Copolymer, Polyvinylacetat, Polyvinylpyrrolidon.

Die nachfolgenden Beispiele sollen die Erfindung näher erläutern, ohne sie jedoch darauf zu beschränken.

Beispiel 1

Coffein-Tabletten mit Copolyvidon (Kollidon® VA 64)

Tablettiermischung (A) bestehend aus 320 g Coffein und 320 g einer formulierten Mischung aus Polyvinylacetat und Polyvinylpyrrolidon im Verhältnis 8:2 (= Kollidon®SR) und 3,2 g Mg-Stearat; Tablettiermischung (B) bestehend aus 320 g Coffein und 320 g einer formulierten Mischung aus Polyvinylacetat und Polyvinylpyrrolidon im Verhältnis 8:2, 80 g Kollidon® VA 64 (Copolymerisat aus Vinylacetat und Vinylpyrrolidon im Verhältnis 6:4) und 3,6 g Mg-Stearat; Tablettiermischung (C) bestehend aus 320 g Coffein und 360 g einer formulierten Mischung aus Polyvinylacetat und Polyvinylpyrrolidon im Verhältnis 8:2, 160 g Kollidon®VA 64 und 4,2 g Mg-Stearat;

Siebung der einzelnen Pulverbestandteile über ein 800 $\mu$m -Sieb, 10-minütiges Vermischen in einem Turbula-Mischer. Die jeweiligen Tabletten (10 mm, rund, biplan mit facettiertem Rand) wurden auf einer Exzenterpresse (Korsch EK0) bei einem Preßdruck von 18kN gepreßt.

Bestimmung der Bruchfestigkeit mit einem Krämer Tabletten-Tester (HAT-TMB), Friabilität am Erweka Friabilator); Freigabeuntersuchung gemäß USP XXIV-Methode an einem Erweka DT80 Freigabegerät, Paddle-Methode, 50 upm, 0 bis 2 h in 0,08N HCl-Medium, danach Umpufferung auf pH 6,8 mit Phosphatpufferlösung.

Tabelle 1: Zusammensetzung der Tablettenchargen [mg]:

| Charge: | A | B | C |
|---|---|---|---|
| | | | |
| Coffein | 160 | 160 | 160 |
| Kollidon SR | 160 | 160 | 180 |
| Kollidon VA64 | - | 40 | 80 |
| Mg-Stearat | 1,6 | 2 | 1,8 |
| | | | |
| Bruchfestigkeit [N] | 295 | 325 | > 325 |
| Friabilität [%] | 0,01 | < 0.01 | < 0,01 |

Tabelle 2

| Zeit [h] | Wirkstofffreisetzung [%] | | |
|---|---|---|---|
| | K.SR 160 mg [A] | K.SR/K.VA 64 160/40 mg [B] | K.SR/K.VA 64 160/80 mg [C] |
| | | | |
| 0 | 0 | 0 | 0 |
| 0,5 | 10,9 | 15,2 | 17,5 |
| 1 | 16,9 | 21,6 | 22,9 |
| 1,5 | 20,7 | 25,4 | 28,0 |
| 2,19 | 24,4 | 29,5 | 32,0 |
| 3 | 29,7 | 35,2 | 37,8 |
| 4 | 33,9 | 38,7 | 41,9 |
| 6 | 40,3 | 44,8 | 51,7 |
| 8 | 46,1 | 51,4 | 61,0 |
| 12 | 55,8 | 64,4 | 74,3 |
| 16 | 64,4 | 72,7 | 83,8 |

Der Zusatz von Kollidon®VA 64 beschleunigt die Freisetzung und verbessert die mechanischen Eigenschaften.

Beispiel 2

Coffein-Tabletten mit Hydroxypropylmethylcellulose (Methocel® K 100 M)

Tablettiermischung (A) Vgl. Bsp. 1. Tablettiermischung (D) bestehend aus 320 g Coffein und 320 g einer formulierten Mischung aus Polyvinylacetat und Polyvinylpyrrolidon im Verhältnis 8:2 (=Kollidon®SR), 20 g Methocel®K 100M und 3,3 g Mg-Stearat; Tablettiermischung (E) bestehend aus 320 g Coffein und 20 g Methocel K 100 M und 1,7 g Mg-Stearat.

Siebung der einzelnen Pulverbestandteile über ein 800 $\mu$m Sieb, 10minütiges Vermischen in einem Turbula-Mischer. Die jeweiligen Tabletten (10 mm, rund, biplan mit facettiertem Rand) wurden auf einer Exzenterpresse (Korsch EK0) bei einem Preßdruck von 18kN gepreßt.

Bestimmung der Bruchfestigkeit mit einem Krämer Tabletten-Tester (HAT-TMB), Friabilität am Erweka Friabilator); Freigabeuntersuchung gemäß USP XXIV-Methode an einem Erweka DT80 Freigabegerät, Paddle-Methode, 50 upm, 0 bis 2 h in 0,08N HCl-Medium, danach Umpufferung auf pH 6,8 mit Phosphatpufferlösung.

Tabelle 3: Zusammensetzung der Tablettenchargen [mg]:

| Charge: | A | D | E |
|---|---|---|---|
| | | | |
| Coffein | 160 | 160 | 160 |
| Kollidon SR | 160 | 160 | - |
| Methocel K100M | - | 10 | 10 |
| Mg-Stearat | 1,6 | 1,65 | 0,85 |
| | | | |
| Bruchfestigkeit [N] | 295 | 305 | 132 |
| Friabilität [%] | 0,01 | 0,01 | 0,18 |

Tabelle 4

| Zeit [h] | Wirkstofffreisetzung [%] | | |
|---|---|---|---|
| | K.SR 160 mg [A] | K.SR/Methocel 160/10 mg [D] | Methocel 10 mg [E] |
| | | | |
| 0 | 0 | 0 | 0 |
| 0,5 | 9,46 | 5,0 | 67,7 |
| 1 | 15,19 | 8,9 | 88,0 |
| 1,5 | 18,22 | 12,5 | 92,7 |
| 2 | 22,03 | 16,1 | 93,2 |
| 3 | 26,61 | 20,7 | 94,0 |
| 4 | 31,65 | 25,6 | - |
| 6 | 39,27 | 33,5 | - |
| 8 | 46,11 | 38,7 | - |
| 12 | 58,10 | 49,5 | - |
| 16 | 67,21 | 56,9 | - |

Schon ein geringer Zusatz von Methocel®K 100 M führt zu einer verlangsamten Freisetzung bei ausgezeichneten mechanischen Eigenschaften. Tabletten nur mit 10 mg Methocel®K 100 M zeigen keine Retardwirkung.

Beispiel 3

Diclofenac-Tabletten mit Hydroxypropylmethylcellulose (Methocel® K 100 M)

Tablettiermischung (F) bestehend aus 200 g Diclofenac-Na und 200 g einer formulierten Mischung aus Polyvinylacetat und Polyvinylpyrrolidon im Verhältnis 8:2 (=Kollidon®SR), 6 g Mg-Stearat; Tablettiermischung (G) bestehend aus 200 g Diclofenac und 200 g einer formulierten Mischung aus Polyvinylacetat und Polyvinylpyrrolidon im Verhältnis 8:2, 40 g Methocel®K 100 M und 6,0 g Mg-Stearat; Tablettiermischung (H) bestehend aus 200 g Diclofenac-Na und 200 g einer formulierten Mischung aus Polyvinylacetat und Polyvinylpyrrolidon im Verhältnis 8:2, 100 g Methocel® K 100 M und 6,0 g Mg-Stearat.
Tablettiermischung (I) bestehend aus 200 g Diclofenac-Na und 200 g Methocel® K 100 M und 6,0 g Mg-Stearat.
Siebung der einzelnen Pulverbestandteile über ein 800 μm Sieb, 10minütiges Vermischen in einem Turbula-Mischer. Die jeweiligen Tabletten (8 mm, rund, biplan mit facettiertem Rand) wurden auf einer Rundläuferpresse (Korsch PH 106) bei einem Preßdruck von 10kN gepreßt.
Bestimmung der Bruchfestigkeit mit einem Krämer Tabletten-Tester (HAT-TMB), Friabilität am Erweka Friabilator); Freigabeuntersuchung gemäß USP XXIV-Methode an einem Erweka DT80 Freigabegerät, Paddle-Methode, 50 upm, 0 bis 16 h in Phosphatpufferlösung pH 6,8.

Tabelle 5: Zusammensetzung der Tablettenchargen [mg]:

| Charge: | F | G | H | I |
|---|---|---|---|---|
| | | | | |
| Diclofenac-Na | 100 | 100 | 100 | 100 |
| Kollidon SR | 100 | 100 | 100 | - |
| Methocel K100M | - | 20 | 50 | 100 |
| Mg-Stearat | 3 | 3 | 3 | 3 |
| | | | | |

(fortgesetzt)

| Charge: | F | G | H | I |
|---|---|---|---|---|
| Bruchfestigkeit [N] | 218 | 244 | 270 | 106 |
| Friabilität [%] | 0,01 | 0,01 | 0,01 | 0,15 |

Tabelle 6

| | Wirkstofffreisetzung [%] | | | |
|---|---|---|---|---|
| Zeit [h] | K.SR 100 mg [F] | K.SR/Methocel 100/20 (mg) [G] | K.SR/Methocel 100/50 mg [H] | Methocel 100 mg [I] |
| | | | | |
| 0 | 0 | 0 | 0 | 0 |
| 0,56 | 5,4 | 5,0 | 3,7 | 33,2 |
| 1 | 11,5 | 10,8 | 9,2 | 61,5 |
| 1,5 | 18,8 | 16,1 | 13,8 | 77,9 |
| 2 | 27,0 | 21,8 | 17,4 | 87,7 |
| 3 | 37,0 | 31,7 | 22,5 | 89,0 |
| 4 | 49,0 | 42,0 | 31,3 | 92,6 |
| 6,12 | 74,1 | 63,0 | 41,0 | 95,5 |
| 8 | 99,8 | 80,0 | 53,3 | 98,3 |
| 12 | 98,9 | 92,8 | 67,0 | 97,9 |
| 16 | 100,0 | 97,4 | 79,8 | 98,5 |

Die Retardwirkung einer formulierten Mischung aus Polyvinylacetat und Polyvinylpyrrolidon läßt sich durch Methocel® K 100 M steigern, obwohl Methocel® alleine nahezu keine Retardwirkung auf Diclofenac besitzt. Die mechanischen Eigenschaften der Kombination sind besser als die der Einzelkomponenten.

Beispiel 4

Coffein-Tabletten mit Methylhydroxyethylcellulose (Tylose® M6)

Tablettiermischung (A) Vgl. Bsp. 1. Tablettiermischung (K) bestehend aus 320 g Coffein und 320 g einer formulierten Mischung aus Polyvinylacetat und Polyvinylpyrrolidon im Verhältnis 8:2 (= Kollidon® SR), 80 g Tylose® M6 und 3,6 g Mg-Stearat.
Siebung der einzelnen Pulverbestandteile über ein 800 µm Sieb, 10-minütiges Vermischen in einem Turbula-Mischer. Die jeweiligen Tabletten (10 mm, rund, biplan mit facettiertem Rand) wurden auf einer Exzenterpresse (Korsch EK0) bei einem Preßdruck von 18kN gepreßt.
Bestimmung der Bruchfestigkeit mit einem Krämer Tabletten-Tester (HAT-TMB), Friabilität am Erweka Friabilator); Freigabeuntersuchung gemäß USP XXIV-Methode an einem Erweka DT80 Freigabegerät, Paddle-Methode, 50 upm, 0 bis 2 h in 0,08N HCl-Medium, danach Umpufferung auf pH 6,8 mit Phosphatpufferlösung.

Tabelle 7: Zusammensetzung der Tablettenchargen [mg]:

| Charge: | A | K |
|---|---|---|
| | | |
| Coffein | 160 | 160 |
| Kollidon SR | 160 | 160 |
| Tylose M6 | - | 40 |

(fortgesetzt)

| Charge: | A | K |
|---|---|---|
| Mg-Stearat | 1,6 | 1,8 |
| | | |
| Bruchfestigkeit [N] | 295 | > 350 |
| Friabilität [%] | 0,01 | < 0,01 |

Tabelle 8

| | Wirkstofffreisetzung [%] | |
|---|---|---|
| Zeit [h] | K.SR 160 mg [A] | K.SR/Tylose 160/40 mg [K] |
| | | |
| 0 | 0 | 0 |
| 0,5 | 10,9 | 5,7 |
| 1 | 16,9 | 10,5 |
| 1,5 | 20,7 | 14,2 |
| 2 | 24,4 | 17,2 |
| 3 | 29,7 | 22,7 |
| 4 | 33,9 | 27,1 |
| 6 | 40,3 | 35,2 |
| 8 | 46,1 | 40,4 |
| 12 | 55,8 | 50,7 |
| 16 | 64,4 | 60,1 |

Der geringe Zusatz von Tylose verlangsamt die Freisetzung und verbessert deutlich die mechanischen Eigenschaften.

Beispiel 5

Coffein-Tabletten mit Stearinsäure

Tablettiermischung (A) Vgl. Bsp. 1. Tablettiermischung (L) bestehend aus 320 g Coffein und 320 g einer formulierten Mischung aus Polyvinylacetat und Polyvinylpyrrolidon im Verhältnis 8:2 (= Kollidon® SR), 40 g Stearinsäure und 3,6 g Mg-Stearat.

Siebung der einzelnen Pulverbestandteile über ein 800 μm Sieb, 10minütiges Vermischen in einem Turbula-Mischer. Die jeweiligen Tabletten (10 mm, rund, biplan mit facettiertem Rand) wurden auf einer Exzenterpresse (Korsch EK0) bei einem Preßdruck von 18kN gepreßt.

Bestimmung der Bruchfestigkeit mit einem Krämer Tabletten-Tester (HAT-TMB), Friabilität am Erweka Friabilator); Freigabeuntersuchung gemäß USP XXIV-Methode an einem Erweka DT80 Freigabegerät, Paddle-Methode, 50 upm, 0 bis 2 h in 0,08N HCl-Medium, danach Umpufferung auf pH 6,8 mit Phosphatpufferlösung.

Tabelle 9: Zusammensetzung der Tablettenchargen [mg]:

| Charge: | A | L |
|---|---|---|
| | | |
| Coffein | 160 | 160 |

(fortgesetzt)

| Charge: | A | L |
|---|---|---|
| Kollidon SR | 160 | 160 |
| Stearinsäure | - | 40 |
| Mg-Stearat | 1,6 | 1,8 |
| | | |
| Bruchfestigkeit [N] | 295 | 274 |
| Friabilität [%] | 0,01 | 0,02 |

Tabelle 10

| | Wirkstofffreisetzung [%] | |
|---|---|---|
| Zeit [h] | K.SR 160 mg [A] | K.SR/Stearinsäure 160/40 mg [L] |
| | | |
| 0 | 0 | 0 |
| 0,5 | 10,9 | 7,3 |
| 1 | 16,9 | 11,5 |
| 1,5 | 20,7 | 14,8 |
| 2 | 24,4 | 17,2 |
| 3 | 29,7 | 21,8 |
| 4 | 33,9 | 24,7 |
| 6 | 40,3 | 30,0 |
| 8 | 46,1 | 34,4 |
| 12 | 55,8 | 43,4 |
| 16 | 64,4 | 49,7 |

Der geringe Zusatz von Stearinsäure verlangsamt die Freisetzung des Wirkstoffes deutlich.

Beispiel 6

Propranolol-Tabletten mit Methacrylsäure-Ethylacrylat-Copolymer (Kollicoat® MAE 100 P)

Tablettiermischung (M) bestehend aus 320 g Propranolol-HCl und 320 g einer formulierten Mischung aus Polyvinylacetat und Polyvinylpyrrolidon im Verhältnis 8:2 (= Kollidon® SR),
und 6,4 g Mg-Stearat; Tablettiermischung (N) bestehend aus 320 g Propranolol-HCl und 320 g einer formulierten Mischung aus Polyvinylacetat und Polyvinylpyrrolidon im Verhältnis 8:2, 80 g Kollicoat® MAE 100 P und 7,2 g Mg-Stearat.
Siebung der einzelnen Pulverbestandteile über ein 800 μm Sieb, 10-minütiges Vermischen in einem Turbula-Mischer. Die jeweiligen Tabletten (10 mm, rund, biplan mit facettiertem Rand) wurden auf einer Rundläuferpresse (Korsch PH 106) bei einem Preßdruck von 18kN gepreßt.
Bestimmung der Bruchfestigkeit mit einem Krämer Tabletten-Tester (HAT-TMB), Friabilität am Erweka Friabilator); Freigabeuntersuchung gemäß USP XXIV-Methode an einem Erweka DT80 Freigabegerät, Paddle-Methode, 50 upm, 0 bis 2 h in 0,08N HCl-Medium, danach Umpufferung auf pH 6,8 mit Phosphatpufferlösung.

Tabelle 11: Zusammensetzung der Tablettenchargen [mg]:

| Charge: | M | N |
|---|---|---|
| | | |
| Propranolol | 160 | 160 |
| Kollidon SR | 160 | 160 |
| Kollicoat MAE 100P | - | 40 |
| Mg-Stearat | 3,2 | 3.6 |
| | | |
| Bruchfestigkeit [N] | 216 | 271 |
| Friabilität [%] | 0,02 | 0,02 |

Tabelle 12

| | Wirkstofffreisetzung [%] | |
|---|---|---|
| Zeit [h] | K.SR 160 mg [M] | K.SR/K. MAE 160/40 mg [N] |
| | | |
| 0 | 0 | 0 |
| 0,5 | 19,4 | 10,0 |
| 1 | 25,3 | 15,5 |
| 1,5 | 31,8 | 18,9 |
| 2 | 38,0 | 22,4 |
| 2,5 | 41,5 | 24,6 |
| 3 | 45,8 | 26,5 |
| 4 | 53,9 | 30,6 |
| 5 | 59,7 | 33,4 |
| 6 | 64,2 | 34,7 |
| 7 | 68,9 | 36,6 |
| 8 | 71,8 | 38,2 |
| 9 | 74,8 | 40,4 |
| 10 | 77,3 | 41,7 |
| 11 | 79,4 | 43,7 |
| 12 | 81,8 | 45,4 |
| 16 | 86,3 | 51,7 |

Der Zusatz von Kollicoat® MAE 100P verbessert die mechanischen Eigenschaften und reduziert die Freisetzung.

**Patentansprüche**

1. Nicht schwimmfähiges orale Darreichungsformen mit retardierter Wirkstofffreisetzung und hoher mechanischer Stabilität, enthaltend

a) einen oder mehrere Wirkstoffe
b) eine vorformulierte Mischung aus Polyvinylacetat und Polyvinylpyrrolidon
c) wasserlösliche Polymeren oder nieder- oder hochmolekulare lipophile Zusatzstoffe
d) sowie weitere, übliche Hilfsstoffe,

wobei das Verhältnis Polyvinylacetat und Polyvinylpyrrolidon 6:4 bis 9:1 beträgt.

2. Orale Darreichungsformen gemäß Anspruch 1, **dadurch gekennzeichnet, daß** eine formulierte Mischung aus Polyvinylacetat und Polyvinylpyrrolidon im Verhältnis 8:2 eingesetzt wird.

3. Orale Darreichungsformen gemäß einem der Ansprüche 1 oder 2, **dadurch gekennzeichnet, daß** es sich bei den Darreichungsformen um Tabletten, Extrudate, Pellets oder Granulate handelt.

4. Orale Darreichungsformen gemäß einem der Ansprüche 1 bis 3, **dadurch gekennzeichnet, daß** ein wasserlösliches oder wasserunlösliches retardierendes Coating auf die orale Darreichungsform aufgebracht wird.

5. Orale Darreichungsformen gemäß einem der Ansprüche 1 bis 4, **dadurch gekennzeichnet, daß** die wasserlöslichen bzw. lipophilen Polymere ausgewählt sind aus der Gruppe der: Polyvinylalkohole, Polyethylenglykole, Polyoxyethylen-Polyoxypropylen-Dlockpolymerisate, Polyvinylpyrrolidone sowie Derivate, Vinylacetat-Vinylpyrrolidon-Copolymere, vorzugsweise Polyethylenglykolen, Polyvinylpyrrolidonen, Vinylacetat-Vinylpyrrolidon-Copolymeren oder Maltodextrinen, sowie Salzen davon.

6. Orale Darreichungsformen gemäß einem der Ansprüche 1 bis 5, **dadurch gekennzeichnet, daß** die wasserlöslichen quellenden Polymere ausgewählt sind aus der Gruppe der: Alginate, Pektine, Galactomannane, Carrageenane, Dextran, Curdlan, Pullulan, Gellan, Chitin, Gelatine, Xanthane, Hemicellulosen, Cellulosederivate wie Methylcellulose, Hydroxypropylmethylcellulose, Hydroxypropylcellulose, Hydroxyethylcellulose, Methylhydroxyethylcellulose, Carboxymethylcellulose, Stärkederivate wie Carboxymethylstärke, abgebaute Stärke, Polyacrylsäure, Polymethacrylsäure, Acrylsäure-Methacrylsäure-Copolymere, sowie Salzen davon.

7. Orale Darreichungsformen gemäß einem der Anspruche 1 bis 6, **dadurch gekennzeichnet, daß** die lipophilen Zusatzstoffe ausgewählt sind aus der Gruppe der: Cellulosederivate wie Ethylcellulose, Celluloseacetat, Celluloseacetatphthalat, Celluloseacetatsuccinat, Hydroxypropylmethylcelluloseacetatphthalat, Hdroxypropylmethylcelluloseacetatsuccinat, Acrylatester-Methacrylatester-Copolymerisate insbesondere Methylmethacrylat-Ethylacrylat-Copolymere, Ammonio-Methacrylate-Copolymer Typ A und Typ B, Methacrylsäure-Acrylsäureester-Copolymere insbesondere Methacrylsäure-Ethylacrylat-Copolymere, Fettalkohole wie Stearylalkohol, Fettsäure wie Stearinsäure, Fettsäureester und Fettalkoholester, Glyceride, Wachse, Lecithin.

8. Orale Darreichungsformen gemäß einem der Ansprüche 1 bis 7, **dadurch gekennzeichnet, daß** sie mittels Direktverpressung, Extrusion, Schmelzextrusion, Pelletierung, Kompaktierung, Feuchtgranulierung hergestellt werden.

9. Orale Darreichungsformen gemäß einem der Ansprüche 1 bis 8, **dadurch gekennzeichnet, daß** als übliche Hilfsstoffe Bindemittel, Streckmittel/Füllstoffe, Sprengmittel, Schmiermittel, Fließmittel, Farbstoffe, Stabilisatoren wie Antioxidantien, Netzmittel, Konservierungsmittel, Formentrennmittel, Aromen und Süßstoffe eingesetzt werden.

10. Orale Darreichungsformen gemäß einem der Ansprüche 1 bis 9, **dadurch gekennzeichnet, daß** die formulierte Mischung aus Polyvinylacetat und Polyvinylpyrrolidon in einem Anteil von 10 bis 80 %, bezogen auf das Tablettengesamtgewicht, vorliegt.

11. Orale Darreichungsformen gemäß einem der Ansprüche 1 bis 10, **dadurch gekennzeichnet, daß** die wasserlöslichen Polymere und/oder die lipophilen Zusatzstoffe in einem Anteil von 1 bis 40 %, bezogen auf das Tablettengesamtgewicht, vorliegen.

12. Orale Darreichungsformen gemäß einem der Ansprüche 1 bis 11, **dadurch gekennzeichnet, daß** als wasserlösliche Polymere Hydroxypropylmethylcellulosen eingesetzt werden.

13. Orale Darreichungsformen gemäß einem der Ansprüche 1 bis 11, **dadurch gekennzeichnet, daß** als wasserlösliche Polymere Polyvinylpyrrolidone oder Vinylacetat-Vinylpyrrolidon-Copolymere eingesetzt werden.

**14.** Orale Darreichungsformen gemäß einem der Ansprüche 1 bis 13, **dadurch gekennzeichnet, daß** es sich um eine Manteltablette mit wirkstoffreichem Kern handelt.

**15.** Orale Darreichungsformen gemäß einem der Ansprüche 1 bis 14, **dadurch gekennzeichnet, daß** sie als Wirkstoffe Nahrungsergänzungs- oder Zusatzstoffe, Vitamine, Mineralstoffe oder Spurenelemente oder pharmazeutische Wirkstoffe enthalten.

**16.** Orale Darreichungsformen gemäß einem der Ansprüche 1 bis 15, **dadurch gekennzeichnet, daß** sie als Wirkstoffe pharmazeutische Wirkstoffe enthalten.

**17.** Darreichungsform gemäß einem der Ansprüche 1 bis 16, **dadurch gekennzeichnet, daß** der pharmazeutische Wirkstoff ausgewählt ist aus der Gruppe der Benzodiazepine, Antihypertensiva, Vitamine, Cytostatika, Anästhetika, Neuroleptika, Antidepressiva, Antibiotika, Antimykotika, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Durchblutungsfördernde Mittel, Diuretika, Diagnostika, Corticoide, Cholinergika, Gallenwegstherapeutika, Antiasthmatika, Broncholytica, Betarezeptorenblocker, Calciumantagonisten, ACE-Hemmer, Arteriosklerosemittel, Antiphlogistika, Antikoagulatia, Antihypotonika, Antihypoglykämika, Antihypertonika, Antifibrinolytika, Antiepileptika,Antiemetika, Antidota, Antidiabetika, Antiarrhythmika, Antianämika, Antiallergika, Anthelmintika, Analgetika, Analeptika, Aldosteronantagonisten, Abmagerungsmittel.

**18.** Arzneimittel zur verzögerten Wirkstofffreisetzung, **dadurch gekennzeichnet, daß** es sich um eine orale Darreichungsform gemäß einem der Ansprüche 1 bis 17 handelt.

**19.** Verwendung der oralen Darreichungsformen gemäß einem der Patentansprüche 1 bis 17 zur Herstellung von Arzneimitteln mit verzögerter Wirkstofffreisetzung.

**20.** Verwendung der oralen Darreichungsformen gemäß einem der Ansprüche 1 bis 17, zur verzögerter Wirkstofffreisetzung von Nahrungsergänzungs- oder Zusatzstoffen, Vitaminen, Mineralstoffen oder Spurenelementen.

**Claims**

**1.** A non-buoyant oral dosage form with delayed release of active ingredient and high mechanical stability, comprising

a) one or more active ingredients
b) a preformulated mixture of polyvinyl acetate and polyvinylpyrrolidone
c) water-soluble polymers or low or high molecular weight lipophilic additives
d) and other conventional excipients,

the ratio of polyvinyl acetate to polyvinylpyrrolidone being from 6:4 to 9:1.

**2.** The oral dosage form according to claim 1, wherein a formulated mixture of polyvinyl acetate and polyvinylpyrrolidone in the ratio 8:2 is employed.

**3.** The oral dosage form according to either of claims 1 or 2, which is a tablet, extrudate, pellet or granulate.

**4.** The oral dosage form according to any of claims 1 to 3, wherein a water-soluble or water-insoluble release-delaying coating is applied to the oral dosage form.

**5.** The oral dosage form according to any of claims 1 to 4, wherein the water-soluble or lipophilic polymers are selected from the group of: polyvinyl alcohols, polyethylene glycols, polyoxyethylene/polyoxypropylene block copolymers, polyvinylpyrrolidones and derivatives, vinyl acetate/vinylpyrrolidone copolymers, preferably polyethylene glycols, polyvinylpyrrolidones, vinyl acetate/vinylpyrrolidone copolymers or maltodextrins, and salts thereof.

6. The oral dosage form according to any of claims 1 to 5, wherein the water-soluble swelling polymers are selected from the group of: alginates, pectins, galactomannans, carrageenans, dextran, curdlan, pullulan, gellan, chitin, gelatin, xanthans, hemicelluloses, cellulose derivatives such as methylcellulose, hydroxypropylmethylcellulose, hydroxypropylcellulose, hydroxyethylcellulose, methylhydroxyethylcellulose, carboxymethylcellulose, starch derivatives such as carboxymethyl starch, degraded starch, polyacrylic acid, polymethacrylic acid, acrylic acid/methacrylic acid copolymers, and salts thereof.

7. The oral dosage form according to any of claims 1 to 6, wherein the lipophilic additives are selected from the group of: cellulose derivatives such as ethylcellulose, cellulose acetate, cellulose acetate phthalate, cellulose acetate succinate, hydroxypropylmethylcellulose acetate phthalate, hydroxypropylmethylcellulose acetate succinate, acrylic ester/methacrylic ester copolymers, in particular methyl methacrylate/ethyl acrylate copolymers, ammoniomethacrylate copolymer type A and type B, methacrylic acid/acrylic ester copolymers, in particular methacrylic acid/ethyl acrylate copolymers, fatty alcohols such as stearyl alcohol, fatty acids such as stearic acid, fatty acid esters and fatty alcohol esters, glycerides, waxes, lecithin.

8. The oral dosage form according to any of claims 1 to 7, which is produced by direct compression, extrusion, melt extrusion, pelleting, compaction, wet granulation.

9. The oral dosage form according to any of claims 1 to 8, wherein binders, extenders/fillers, disintegrants, lubricants, flow regulators, dyes, stabilizers such as antioxidants, wetting agents, preservatives, release agents, flavorings and sweeteners are employed as conventional excipients.

10. The oral dosage form according to any of claims 1 to 9, wherein the formulated mixture of polyvinyl acetate and polyvinylpyrrolidone is present in a proportion of from 10 to 80% based on the total weight of the tablet.

11. The oral dosage form according to any of claims 1 to 10, wherein the water-soluble polymers and/or the lipophilic additives are present in a proportion of from 1 to 40% based on the total weight of the tablet.

12. The oral dosage form according to any of claims 1 to 11, wherein hydroxypropylmethylcelluloses are employed as water-soluble polymers.

13. The oral dosage form according to any of claims 1 to 11, wherein polyvinylpyrrolidones or vinyl acetate/vinylpyrrolidone copolymers are employed as water-soluble polymers.

14. The oral dosage form according to any of claims 1 to 13, which is a press-coated tablet whose core is rich in active ingredient.

15. The oral dosage form according to any of claims 1 to 14, which comprises as active ingredients food supplements or additives, vitamins, minerals or trace elements or active pharmaceutical ingredients.

16. The oral dosage form according to any of claims 1 to 15, which comprises active pharmaceutical ingredients as active ingredients.

17. The dosage form according to any of claims 1 to 16, wherein the active pharmaceutical ingredient is selected from the group of benzodiazepines, antihypertensives, vitamins, cytostatics, anesthetics, neuroleptics, antidepressants, antibiotics, antimycotics, fungicides, chemotherapeutics, urologicals, platelet aggregation inhibitors, sulfonamides, spasmolytics, hormones, immunoglobulins, sera, thyroid therapeutics, psychopharmaceuticals, antiparkinson agents and other antihyperkinetics, ophthalmologicals, neuropathy products, calcium metabolism regulators, muscle relaxants, lipid-lowering agents, liver therapeutics, coronary agents, cardiac agents, immunotherapeutics, regulatory peptides and their inhibitors, hypnotics, sedatives, gynecologicals, antigout agents, fibrinolytics, enzyme products and transport proteins, enzyme inhibitors, emetics, perfusion promoters, diuretics, diagnostics, corticoids, cholinergics, biliary therapeutics, antiasthmatics, bronchospasmolytics, beta-receptor blockers, calcium channel blockers, ACE inhibitors, arteriosclerosis remedies, antiinflammatory agents, anticoagulants, antihypotensives, antihypoglycemics, antifibrinolytics, antiepileptics, antiemetics, antidotes, antidiabetics, antiarrhythmics, antianemics, antiallergics, anthelmintics, analgesics, analeptics, aldosterone antagonists, weight-reducing agents.

18. The drug for delayed release of active ingredient, which is an oral dosage form according to any of claims 1 to 17.

**19.** The use of the oral dosage forms according to any of claims 1 to 17 for producing drugs with delayed release of active ingredient.

**20.** The use of the oral dosage forms according to any of claims 1 to 17 for delayed release of active ingredients which are food supplements or additives, vitamins, minerals or trace elements.

## Revendications

**1.** Formes d'administration orale non flottables à libération retardée de la substance active et à stabilité mécanique élevée, contenant

   a) une ou plusieurs substances actives,
   b) un mélange préformulé d'acétate de polyvinyle et de polyvinylpyrrolidone,
   c) des polymères solubles dans l'eau ou des additifs lipophiles à bas ou haut poids moléculaire,
   d) ainsi que d'autres adjuvants courants,

   le rapport entre acétate de polyvinyle et polyvinylpyrrolidone étant de 6/4 à 9/1.

**2.** Formes d'administration orale suivant la revendication 1, **caractérisées en ce qu'**on met en oeuvre un mélange formulé d'acétate de polyvinyle et de polyvinylpyrrolidone dans un rapport de 8/2.

**3.** Formes d'administration orale suivant l'une des revendications 1 et 2, **caractérisées en ce que**, pour ce qui concerne les formes d'administration, il s'agit de comprimés, d'extrudés, de pellets ou de granules.

**4.** Formes d'administration orale suivant l'une des revendications 1 à 3, **caractérisées en ce qu'**un revêtement retardateur soluble ou insoluble dans l'eau est appliqué sur la forme d'administration orale.

**5.** Formes d'administration orale suivant l'une des revendications 1 à 4, **caractérisées en ce que** les polymères solubles dans l'eau ou respectivement lipophiles sont choisis parmi le groupe des alcools polyvinyliques, des polyéthylèneglycols, des polymères blocs de polyoxyéthylène-polyoxypropylène, des polyvinylpyrrolidones ainsi que de leurs dérivés, des copolymères d'acétate de vinyle-vinylpyrrolidone, de préférence des polyéthylèneglycols, des polyvinylpyrrolidones, des copolymères d'acétate de vinyle-vinylpyrrolidone ou des maltodextrines, ainsi que de leurs sels.

**6.** Formes d'administration orale suivant l'une des revendications 1 à 5, **caractérisées en ce que** les polymères gonflants, solubles dans l'eau, sont choisis parmi le groupe des alginates, des pectines, des galactomannanes, des carragheenanes, du dextrane, du curdlane, du pullulane, du gellane, de la chitine, des gélatines, des xanthanes, des hémicelluloses, des dérivés de cellulose tels que de la méthylcellulose, de l'hydroxypropylméthylcellulose, de l'hydroxypropylcellulose, de l'hydroxyéthylcellulose, de la méthylhydroxyéthylcellulose, de la carboxyméthylcellulose, des dérivés d'amidon, tels que de l'amidon carboxyméthylique, de l'amidon dégradé, de l'acide polyacrylique, de l'acide polyméthacrylique, des copolymères d'acide acrylique-acide méthacrylique, ainsi que de leurs sels.

**7.** Formes d'administration orale suivant l'une des revendications 1 à 6, **caractérisées en ce que** les additifs lipophiles sont choisis parmi le groupe des dérivés de cellulose, tels que de l'éthylcellulose, de l'acétate de cellulose, de l'acétophtalate de cellulose, de l'acétosuccinate de cellulose, de l'acétophtalate d'hydroxypropylméthylcellulose, de l'acétosuccinate d'hydroxypropylméthylcellulose, des copolymères d'ester acrylique-ester méthacrylique, en particulier des copolymères de méthacrylate de méthyle-acrylate d'éthyle, du copolymère ammonio-méthacrylique de type A et de type B, des copolymères d'acide méthacrylique et d'acrylate, en particulier des copolymères d'acide méthacrylique-acrylate d'éthyle, des alcools gras, tels que de l'alcool stéarylique, des acides gras, tels que de l'acide stéarique, des esters d'acides gras et des esters d'alcools gras, des glycérides, des cires, de la lécithine.

**8.** Formes d'administration orale suivant l'une des revendications 1 à 7, **caractérisées en ce qu'**elles sont préparées au moyen d'une compression directe, d'une extrusion, d'une extrusion à l'état fondu, d'une formation de pellets, d'un compactage, d'une granulation à l'état humide.

**9.** Formes d'administration orale suivant l'une des revendications 1 à 8, **caractérisées en ce que**, comme adjuvants courants, on met en oeuvre des liants, des diluants/charges, des agents de libération, des lubrifiants, des agents d'écoulement, des colorants, des stabilisants, tels que des antioxydants, des agents mouillants, des agents de conservation, des agents de démoulage, des arômes et des substances sucrantes.

**10.** Formes d'administration orale suivant l'une des revendications 1 à 9, **caractérisées en ce que** le mélange formulé à base d'acétate de polyvinyle et de polyvinylpyrrolidone se présente en une fraction de 10 à 80 % par rapport au poids total des comprimés.

**11.** Formes d'administration orale suivant l'une des revendications 1 à 10, **caractérisées en ce que** les polymères solubles dans l'eau et/ou les additifs lipophiles se présentent en une fraction de 1 à 40 % par rapport au poids total des comprimés.

**12.** Formes d'administration orale suivant l'une des revendications 1 à 11, **caractérisées en ce que**, comme polymères solubles dans l'eau, on met en oeuvre des hydroxypropylméthylcelluloses.

**13.** Formes d'administration orale suivant l'une des revendications 1 à 11, **caractérisées en ce que**, comme polymères solubles dans l'eau, on met en oeuvre des polyvinylpyrrolidones ou des copolymères d'acétate de vinyle-vinylpyrrolidone.

**14.** Formes d'administration orale suivant l'une des revendications 1 à 13, **caractérisées en ce qu'**il s'agit d'un comprimé à enveloppe présentant un noyau riche en substance active.

**15.** Formes d'administration orale suivant l'une des revendications 1 à 14, **caractérisées en ce qu'**elles contiennent, comme substances actives, des compléments ou suppléments nutritifs, des vitamines, des substances minérales ou des oligo-éléments ou des substances actives pharmaceutiques.

**16.** Formes d'administration orale suivant l'une des revendications 1 à 15, **caractérisées en ce qu'**elles contiennent, comme substances actives, des substances actives pharmaceutiques.

**17.** Forme d'administration suivant l'une des revendications 1 à 16, **caractérisée en ce que** la substance active pharmaceutique est choisie parmi le groupe des benzodiazépines, des antihypertenseurs, des vitamines, des cytostatiques, des anesthésiques, des neuroleptiques, des antidépresseurs, des antibiotiques, des antimycotiques, des fongicides, des substances chimiothérapiques, des agents urologiques, des inhibiteurs d'agrégation des thrombocytes, des sulfamides, des spasmolytiques, des hormones, des immunoglobulines, des sérums, des substances thérapeutiques pour la glande thyroïde, des substances psychopharmaceutiques, des agents antiparkinsoniens et d'autres antihypercinétiques, des substances ophtalmiques, des préparations pour des neuropathies, des agents régulateurs du métabolisme du calcium, des relaxants musculaires, des narcotiques, des réducteurs de lipide, des substances thérapeutiques pour le foie, des agents coronariens, des substances cardiaques, des substances immunothérapeutiques, des peptides régulateurs et leurs inhibiteurs, des hypnotiques, des sédatifs, des produits gynécologiques, des produits contre la goutte, des fibrinolytiques, des préparations enzymatiques et des protéines de transport, des inhibiteurs enzymatiques, des émétiques, des agents favorisant l'irrigation sanguine, des diurétiques, des produits de diagnostic, des corticoïdes, des agents cholinergiques, des substances thérapeutiques pour les voies biliaires, des antiasthmatiques, des broncholytiques, des agents bloquant les bêta-récepteurs, des antagonistes du calcium, des inhibiteurs ACE, des agents contre l'artériosclérose, des antiphlogistiques, des anticoagulants, des antihypnotiques, des antihypoglycémiques, des antihypertoniques, des antifibrinolytiques, des antiépileptiques, des antiémétiques, des antidotes, des agents antidiabète, des antiarythmiques, des antianémiques, des antiallergiques, des anthelminthiques, des analgésiques, des analeptiques, des antagonistes de l'aldostérone, des agents amaigrissants.

**18.** Médicament pour la libération retardée de substances actives, **caractérisé en ce qu'**il s'agit d'une forme d'administration orale suivant l'une des revendications 1 à 17.

**19.** Utilisation des formes d'administration orale suivant l'une des revendications 1 à 17, pour la fabrication de médicaments à libération retardée de substances actives.

**20.** Utilisation des formes d'administration orale suivant l'une des revendications 1 à 17, pour la libération retardée de compléments ou suppléments nutritifs, de vitamines, de matières minérales ou d'oligo-éléments.